(19) Europäisches Patentamt
European Patent Office
Office européen des brevets

(11) EP 1 913 958 B1

(12) **EUROPEAN PATENT SPECIFICATION**

(45) Date of publication and mention of the grant of the patent:
**23.12.2009 Bulletin 2009/52**

(51) Int Cl.:
***A61K 39/395*** *(2006.01)* ***A61K 31/337*** *(2006.01)*
***A61P 35/00*** *(2006.01)*

(21) Application number: **06291264.7**

(22) Date of filing: **03.08.2006**

# (54) Antitumor compositions containing acetylcyclopropyl docetaxel and trastuzumab

Antitumorale Zusammensetzungen enthaltend Acetylcyclopropyl Docetaxel und Trastuzumab

Compositions antitumorales contenant acetylcyclopropyl docetaxel et trastuzumab

(84) Designated Contracting States:
**FR**

(43) Date of publication of application:
**23.04.2008 Bulletin 2008/17**

(73) Proprietor: **Sanofi-Aventis**
**75013 Paris (FR)**

(72) Inventors:
- **Vrignaud, Patricia**
  **77380 Combs La Ville (FR)**
- **Fromond, Claudia**
  **77930 Fleury en Bière (FR)**
- **Bissery, Marie-Christine**
  **94400 Vitry-sur-Seine (FR)**

(74) Representative: **Le Pennec, Magali**
**Sanofi-Aventis**
**20 Avenue Raymond Aron**
**92165 Antony Cedex (FR)**

(56) References cited:
**EP-A1- 0 577 083** **US-A1- 2002 031 505**
**US-B1- 7 041 292**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 913 958 B1

## Description

The present invention relates to combinations of acetylcyclopropyl docetaxel and trastuzumab known under the trade-name Herceptin which are therapeutically useful in the treatment of breast cancer.

The structure of acetocyclopropyl taxotere is as follows:

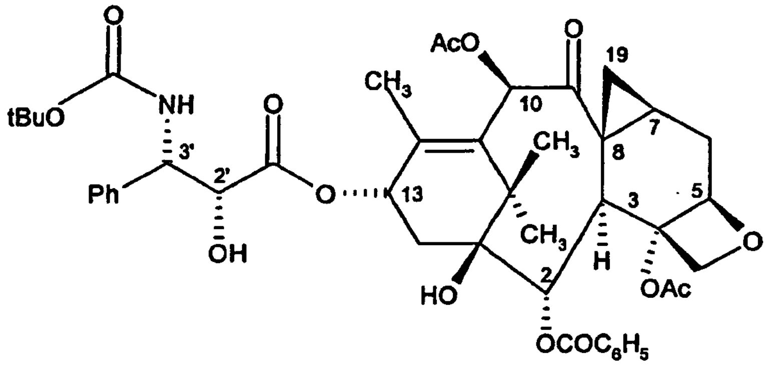

Generally, the doses used, which depend on factors distinctive to the subject to be treated, are between 1 and 100 mg/kg administered intraperitoneally or between 1 and 100 mg/kg administered intravenously.

The monoclonal antibody against ErbB2, also known as trastuzumab is described in EP 153114 as a murine antibody which binds a human breast cancer antigen that is also bound by a reference antibody selected from a list of hybridomas, this list includes ATCC HB 8488 (260F9), ATCC HB 8490 (113F1), ATCC HB 8486 (266B2), ATCC HB 8484C11), ATCC HB 8697 (33F8), ATCC HB 8485 (317G5), ATCC HB 8696 ( 520C9) ATCC HB 8662 (260F9-1C9). Herceptin is produced by a genetically engineered Chinese Hamster Ovary (CHO) cell line, grown in large scale that secretes the antibody into the culture medium. The antibody is purified from the CHO culture media using standard chromatography and filtration methods. The HERCEPTIN is product which is registered nearly everywhere in the world.

It has now been found, and this for invention, that the efficacy of acetylcyclopropyl docetaxel may be considerably improved when they are administered in combination with at least one substance which is therapeutically useful in anticancer treatments and has a mechanism identical to or different from this of taxane derivatives and which is limited in the present invention to trastuzumab or HERCEPTIN.

Moreover, since the activity of the products depends on the doses used, it is possible to use lower doses and to increase the activity while decreasing the toxicity phenomena or delaying their onset by combining growth factors of the haematopoietic type such as G-CSF or GM-CSF or certain interleukins with acetylcyclopropyl docetaxel, or their combinations with other therapeutically active substances.

The improved efficacy of a combination according to the invention may be demonstrated by determination of the therapeutic synergy. A combination manifests therapeutic synergy if it is therapeutically superior to each of the constituents used alone at its maximum tolerated dose.

To demonstrate the efficacy of a combination, it may be necessary to compare the maximum tolerated dose of the combination with the maximum tolerated dose of each of the separate constituents in the study in question. This efficacy may be quantified, for example, by the $\log_{10}$ cells killed, which is determined according to the following formula:

$$\log_{10} \text{ cells killed} = \text{T-C (days)}/3.32\times T_d$$

in which T - C represents the tumor growth delay, which is the mean time in days for the tumors of the treated group (T) and the tumors of the treated group (C) to have reached a predetermined value (1 g for example), and $T_d$ represents the time in days needed for the volume of the tumor to double in the control animals [T.H. Corbett et al., Cancer, 40, 2660-2680 (1977); F.M. Schabel et al., Cancer Drug Development, Part B, Methods in Cancer Research, 17, 3-51, New York, Academic Press Inc. (1979)]. A product is considered to be active if $\log_{10}$ cells killed is greater than or equal to 0.7. A product is considered to be very active if $\log_{10}$ cells killed is greater than 2.8.

The combination, used at its own maximum tolerated dose, in which each of the constituents will be present at a dose generally not exceeding its maximum tolerated dose, will manifest therapeutic synergy when the $\log_{10}$ cells killed is greater than the value of the $\log_{10}$ cells killed of the best constituent when it is administered alone. Even more it is considered more synergistic when the difference in the $\log_{10}$ cells killed is greater than 1 compared to each of the

compound used alone

The efficacy of the combinations on solid tumors may be determined experimentally in the following manner :

The animals subjected to the experiment, generally mice, are subcutaneously grafted bilaterally with 30 to 60 mg of a tumor fragment on day 0. The animals bearing tumors are mixed before being subjected to the various treatments and controls. In the case of treatment of advanced tumors, tumors are allowed to develop to the desired size, animals having insufficiently developed tumors being eliminated. The selected animals are distributed at random to undergo the treatments and controls. Animals not bearing tumors may also be subjected to the same treatments as the tumor-bearing animals in order to be able to dissociate the toxic effect from the specific effect on the tumor. Chemotherapy generally begins from 3 to 22 days after grafting, depending on the type of tumor, and the animals are observed every day. The different animal groups are weighed 3 or 4 times a week until the maximum weight loss is reached, and the groups are then weighed at least once a week until the end of the trial.

The tumors are measured 2 or 3 times a week until the tumor reaches approximately 2 g, or until the animal dies if this occurs before the tumor reaches 2 g. The animals are autopsied when sacrificed.

The antitumor activity is determined in accordance with the different parameters recorded.

For a study of the combinations on leukemias the animals are grafted with a particular number of cells, and the antitumour activity is determined by the increase in the survival time of the treated mice relative to the controls. The product is considered to be active if the increase in survival time is greater than 27%, and is considered to be very active if it is greater than 75% in the case of P388 leukemias.

The results obtained with combinations of acetylpropyl docetaxel and Herceptin, the combinations being used at their optimum dose, are given as example in the following table.

Combinations of acetocyclopropyl taxotere and Herceptin were evaluated in mice bearing s.c. transplantable tumors. The tumor model used to evaluate each drug combination was selected, in general, on the basis of its responsiveness to each of the agents when used as monotherapy. Using i.v. intermittent schedules, full dose response trials were conducted for each single agent and each combination. In the present case the selected tumor was a Human breast carcinoma UISO-BCA-1 derived from a malignant pleural effusion. The primary tumor was well-differenciated infiltrating ductal carcinoma of the breast. Radiotherapy post masectomy was practiced. A chemotherapy 3 years after to treat a left supraclavicular mass was done (10 courses with cytoxan, methotrexate, 5-Fu and Tamoxifen). Tumor cells were sampled during disease progression one month post therapy *(Mehta et al, 1992).* This tumor model was established in nude mice from a $2.10^7$ cell implant, and maintained by serial sc passage as tumor fragment. Using Flow cytometry, the UISO-BCA-1 tumors express HER-2 but not PgP (mdr).

The xenograft was resistant to docetaxel, sensitive to acetylcyclopropyl docetaxel and to 5-Fluorouracil, and was poorly sensitive to doxorubicin, cyclophosphamide and vincristine.

The study was scheduled as a 3-arm dose-response :

- acetyl cyclopropyl docetaxel alone (52.1 - 12.4 mg/kg/iv inj, day 17, 21, 25)
- Herceptin alone (40 - 2.5 mg/kg/ sc inj, day 17, 21, 25, 29)
- Combination with a simultaneous injection of both agents.

The following end points have been used:

- Toxicity was declared at dosages inducing >20% body weight loss or >10 % drug death,
- The Highest NonToxic Dose (HNTD) was determined by the log cell kill = (T-C) / [3.32 x (tumor doubling time in days)]

(T meaning the time of the treated mice to reach 1 g and C the time (26.2 days) of the control mice to reach the same size). No antitumor activity was declared for log cell kill < 0.7, and the treatment was declared highly active for log cell kill > 2.8

- Response rate: Partial Regressions (PR) correspond to regression > 50 % initial tumor burden, and Complete Regressions (CR) to regression below the limit of palpation.

- Therapeutic Synergism: A combination has therapeutic synergism if it is more active than either agent alone (by at least 1 log cell kill).

**Combination of acetocyclopropyl taxotere and Herceptin in human mammary UISO-BCA-1 xenografted in nude mice**

| Dosage in mg/kg/day (total dose in mg/kg) | | Drug deaths | % bwl at nadir | (T-C) days | log cell kill | CR | TFS d121 |
|---|---|---|---|---|---|---|---|
| iv aceto-cyclopropyl taxotere (day 17, 21,25) | sc Herceptin (day 17, 21, 25, 29) | | | | | | |
| 52.1 (156.3) | - | 2/8 | 7.2 | - | - | - | - |
| **32.3 (96.9)** | - | **0/8** | **5.2** | **20.8** | **1.3** | **0/8** | **0/8** |
| 20.0 (60.0) | - | 0/8 | 0.5 | 6.0 | 0.4 | 0/8 | 0/8 |
| 12.4 (37.2) | - | 0/8 | 0.5 | 1.8 | 0.1 | 0/8 | 0/8 |
| - | **40.0 (160.0)** | **0/7** | **0.5** | **0.6** | **0.0** | **0/7** | **0/7** |
| - | 25.0 (100.0) | 0/8 | +13.8 | 0.6 | 0.0 | 0/8 | 0/8 |
| - | 10.0 (40.0) | 0/8 | +14.4 | 0.2 | 0.0 | 0/8 | 0/8 |
| - | 2.5 (10.0) | 0/8 | +11.1 | 1.4 | 0.1 | 0/8 | 0/8 |
| 52.1 (156.3) | 40.0 (160.0) | 4/8 | 14.9 | - | - | - | - |
| 32.3 (96.9) | 40.0 (160.0) | 2/8 | 4.7 | - | - | - | - |
| 32.3 (96.9) | 25.0 (100.0) | 0/8 | 5.9 | 53.9 | 3.3 | 6/8 | 0/8 |
| 32.3 (96.9) | 10.0 (40.0) | 1/8 | 5.5 | - | - | - | - |
| **32.3 (96.9)** | **2.5 (10.0)** | **0/8** | **3.8** | **43.8** | **2.6** | **6/8** | **2/8** |
| 20.0 (60.0) | 40.0 (160.0) | 0/8 | 0.5 | 24.2 | 1.5 | 0/8 | 0/8 |
| 20.0 (60.0) | 25.0 (100.0) | 0/8 | 2.5 | 34.2 | 2.1 | 0/8 | 0/8 |
| 20.0 (60.0) | 10.0 (40.0) | 0/8 | 2.9 | 25.4 | 1.5 | 1/8 | 1/8 |
| 20.0 (60.0) | 2.5 (10.0) | 0/8 | 4.7 | 34.6 | 2.1 | 2/8 | 0/8 |
| 12.4 (37.2) | 40.0 (160.0) | 0/8 | 3.7 | 21.6 | 1.3 | 0/8 | 0/8 |
| 12.4 (37.2) | 25.0 (100.0) | 0/8 | 0.9 | 9.5 | 0.6 | 0/8 | 0/8 |
| 12.4(37.2) | 10.0(40.0) | 0/8 | 1.3 | 5.0 | 0.3 | 0/8 | 0/8 |
| 12.4 (37.2) | 2.5 (10.0) | 0/8 | +5.6 | 3.7 | 0.2 | 0/8 | 0/8 |

Tumor doubling time = 5.0 days. Median tumor burden per group = 125 mg.
Time to reach 1g in controls = 26.2 days.
Abbreviations used: bwl = body weight loss, T-C = tumor growth delay, CR = complete regressions

This combination was synergistic, with a greater activity by more than one log cell kill, than the one observed for the best single agent, acetyl cyclopropyl docetaxel. In this experiment, it is important to note that the highest non-toxic dose of acetyl cyclopropyl docetaxel (32.3 mg/kg per injection, total dose 96.9 mg/kg) given with the lowest dose of Herceptin (2.5 mg/kg/day, total dose 10 mg/kg) was synergistic with a log cell kill of 2.6 compared to 1.3 with the acetyl cyclopropyl docetaxel alone used at its highest non toxic dose. In addition, 6/8 complete regressions and 2/8 long-term tumor-free survivors were achieved by the combination compared to no regression for acetyl cyclopropyl docetaxel alone. A good antitumor activity was observed at a 7 doses level in the combination.

In particular, the combinations can afford the advantage of being able to employ the constituents at considerably lower doses than those at which they are used alone.

The above-included table summarizes the therapeutic response and highest non toxic dose of each arm of the study, the single agents and the combination.

The present invention also relates, therefore, to pharmaceutical compositions containing the combinations according to the invention.

The constituents of which the combination are composed may be administered simultaneously, semi-simultaneously, separately, or spaced out over a period of time so as to obtain the maximum efficacy of the combination; it being possible for each administration to vary in its duration from a rapid administration to a continuous perfusion.

As a result, for the purposes of the present invention, the combinations are not exclusively limited to those

which are obtained by physical association of the constituents, but also to those which permit a separate administration, which can be simultaneous or spaced out over a period of time.

The compositions according to the invention are preferably compositions which can be administered parentally. However, these compositions may be administered orally, subcutaneously or intraperitoneally in the case of localized regional therapies.

The compositions for parental administration are generally pharmaceutically acceptable, sterile solutions or suspensions which may optionally be prepared as required at the time of use. For the preparation of non-aqueous solutions or suspensions, natural vegetable oils such as olive oil, sesame oil or liquid petroleum or injectable organic esters such as ethyl oleate may be used. The sterile aqueous solutions can consist of a solution of the product in water. The aqueous solutions are suitable for intravenous administration provided the pH is appropriately adjusted and the solution is made isotonic, for example with a sufficient amount of sodium chloride or glucose. The sterilization may be carried out by heating or by any other means which does not adversely affect the composition. The combinations may also take the form of liposomes or the form of an association with carriers as cyclodextrins or polyethylene glycols.

The compositions for oral, subcutaneous or intraperitoneal administration are preferably aqueous suspensions or solutions.

In the combinations according to the invention, the application of the constituents of which may be simultaneous, separate or spaced out over a period of time, it is especially advantageous for the amount of taxane derivative to represent from 10 to 90% by weight of the combination, it being possible for this content to vary in accordance with the nature of the associated substance, the efficacy sought and the nature of the cancer to be treated.

The combinations according to the invention are especially useful in the treatment of cancers of the breast. They are mainly useful for treating breast cancers resistant to the commonly used taxoids such as Taxol or Taxotere. There are more preferably used to treat breast cancers resistant to Taxol and/or Taxotere.

## Claims

1. A pharmaceutical combination comprising the compound of formula:

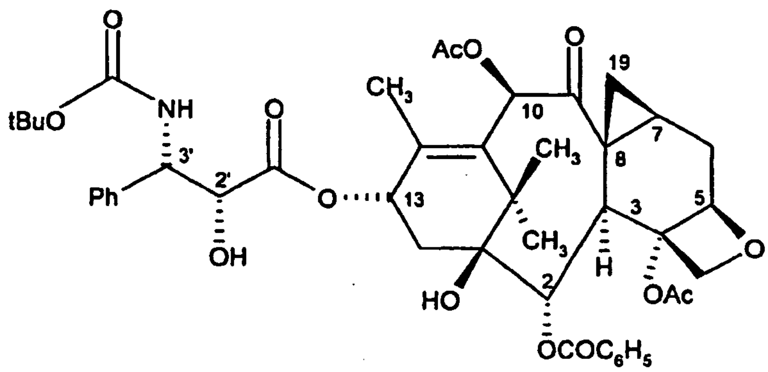

and trastuzumab. for the treatment of breast cancer.

2. The pharmaceutical combination according to claim 1, further comprising growth factors of the haematopoietic type.

3. Use of the compound of formula

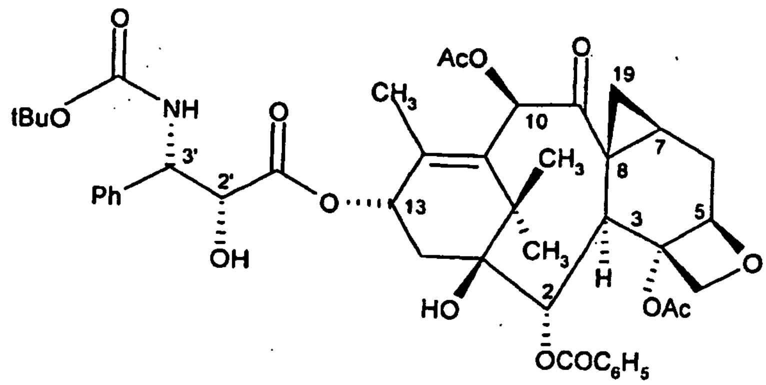

and trastuzumab for the preparation of a pharmaceutical combination according to claim 1 for the treatment of breast cancers resistant to taxoids.

## Patentansprüche

1. Pharmazeutische Kombination, enthaltend die Verbindung der Formel

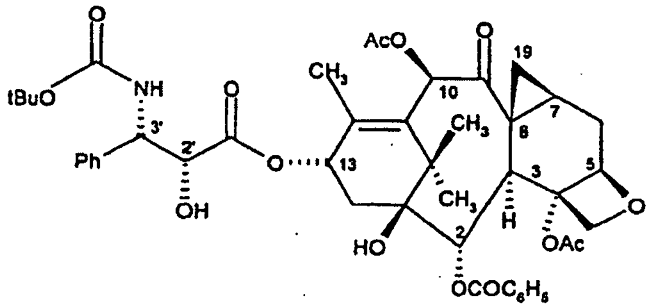

und Trastuzumab zur Behandlung von Brustkrebs.

2. Pharmazeutische Kombination nach Anspruch 1, weiterhin enthaltend Wachstumsfaktoren des hämatopoietischen Typs.

3. Verwendung der Verbindung der Formel

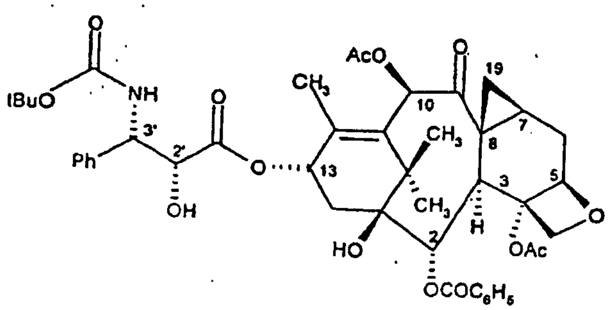

und Trastuzumab zur Herstellung einer pharmazeutischen Kombination nach Anspruch 1 zur Behandlung von taxoidresistentem Brustkrebs.

## Revendications

1. Combinaison pharmaceutique comprenant le composé de formule :

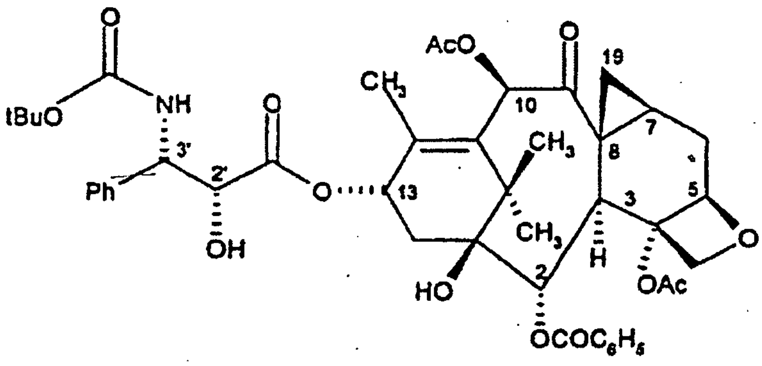

et le trastuzumab, destiné au traitement du cancer du sein.

2. Combinaison pharmaceutique selon la revendication 1, comprenant en outre des facteurs de croissance de type hématopoïétique.

3. Utilisation du composé de formule

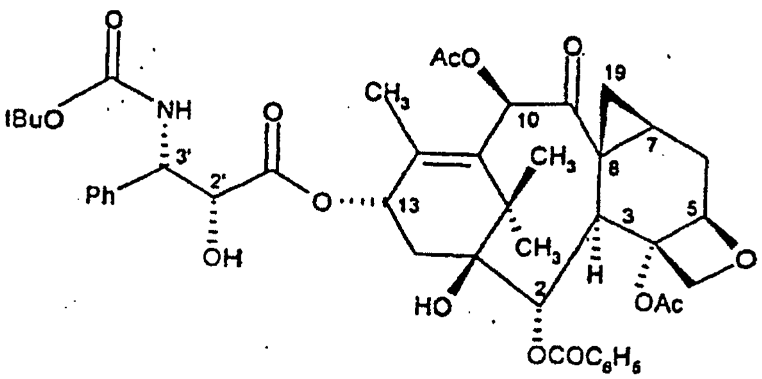

et du trastuzumab pour la préparation d'une combinaison pharmaceutique selon la revendication 1 destinée au traitement des cancers du sein résistants aux taxoïdes.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 153114 A


### Non-patent literature cited in the description


- **T.H. CORBETT et al.** *Cancer,* 1977, vol. 40, 2660-2680
- Methods in Cancer Research. **F.M. SCHABEL et al.** Cancer Drug Development. Academic Press Inc, 1979, vol. 17, 3-51